# EUROPEAN PATENT SPECIFICATION

(11) **EP 1 556 475 B1**
(45) Date of publication and mention of the grant of the patent: **21.11.2007**
(21) Application number: 03798662.7
(22) Date of filing: 11.06.2003
(51) Int. Cl.: C12G 3/02, C12P 7/06, C12N 9/20, C12N 9/18

(54) **FERMENTATION METHODS AND COMPOSITIONS**
FERMENTATIONSVERFAHREN UND -ZUSAMMENSETZUNGEN
PROCEDES ET COMPOSITIONS DE FERMENTATION

(30) Priority: 26.09.2002 US 413730 P
(43) Date of publication of application: 27.07.2005
(73) Proprietor: Novozymes North America, Inc., Franklinton, NC 27515 (US)
(72) Inventor: GRICHKO, Varvara, Raleigh, NC 27614 (US)
(74) Representative: Hansen, Rasmus Rune
(86) International application number: PCT/US2003/018566
(87) International publication number: WO 2004/029193

(56) References cited:
- WO-A-99/49011
- WO-A1-96/13580
- WO-A2-95/21240
- US-A- 5 084 385
- US-A- 5 208 054
- PATENT ABSTRACTS OF JAPAN vol. 011, no. 251 (C-440), 14 August 1987 (1987-08-14) & JP 62 055069 A (AOTO SHOTEN:KK), 10 March 1987 (1987-03-10)
- PATENT ABSTRACTS OF JAPAN vol. 007, no. 039 (C-151), 17 February 1983 (1983-02-17) & JP 57 189638 A (YAKULT HONSHA KK), 22 November 1982 (1982-11-22)

## Description

### FIELD OF THE INVENTION

The present invention relates to enzymatic methods and compositions for producing fermentation products, including, methods and compositions for improving yeast performance during fermentation processes.

### BACKGROUND OF THE INVENTION

Fermentation processes are used for making a vast number of commercial products, including alcohols (e.g., ethanol, methanol, butanol); organic acids (e.g., citric acid, acetic acid, itaconic acid, lactic acid, gluconic acid); ketones (e.g., acetone); amino acids (e.g., glutamic acid); gases (e.g., H₂ and CO₂), and more complex compounds, including, for example, antibiotics (e.g., penicillin and tetracycline); enzymes; vitamins (e.g., riboflavin, B₁₂, beta-carotene); hormones, and other compounds which are difficult to produce synthetically. Fermentation processes are also commonly used in the consumable alcohol (e.g., beer and wine), dairy (e.g., in the production of yogurt and cheese), leather, and tobacco industries. The application of enzymes in fermentation processes are well-known and uses of esterases, such as lipases, or phospolipases, are disclosed in WO 99/49011, JP62055069, JP 5789638 and US5,208,054.
There is a need for further improvement of fermentation processes and for improved processes which include a fermentation step.

### SUMMARY OF THE INVENTION

The present invention provides methods and compositions for producing a fermentation product. The present invention also provides methods and compositions for improving yeast performance during fermentation processes, including the rate and/or yield of the fermentation process. The present invention also provides improved methods and compositions for producing ethanol.

One aspect of the present invention relates to a fermentation process in which at least one esterase enzyme is applied in a fermentation process, wherein said at least one esterase enzyme comprises a cutinase. In a preferred embodiment, the invention comprises contacting a fermenting microorganism or a media used in fermentation with at least one esterase enzyme, wherein said at least one esterase enzyme comprises a cutinase. The esterase may be applied during fermentation and/or the esterase may be applied before fermentation, such as, during propagation of the fermenting microorganisms.

Although not limited to any one theory of operation, the application of esterase enzymes in the fermentation processes according to the present invention, wherein said at least one esterase enzyme comprises a cutinase, is believed to promote a change the lipid composition/concentration inside and/or outside of the fermenting microorganism or in the cell membrane of the fermenting microorganism to result in an improvement in the movement of solutes into and/or out of the fermenting microorganisms during fermentation and/or to provide more metabolizable energy sources, such as, e.g., by converting components of the fermentation substrate or fermentation media, such as, oil from a corn substrate, to components useful to the fermenting microorganisms, e.g., unsaturated fatty acids and glycerol.

In a preferred embodiment of this aspect of the present invention, the at least one esterase, whichat least one esterase comprises a cutinase, furthermore comprises a lipolytic enzyme, more preferably, a lipase. In yet another preferred embodiment, the at least one esterase, which at least one esterase comprises a cutinase, furthermore comprises a phospholipase. In yet another preferred embodiment, the at least one esterase, which at least one esterase comprises a cutinase, furthermore comprises a lipase, a phospholipase and/or combinations thereof.

In a preferred embodiment, the fermentation processes of the present invention are used in combination with a liquefaction process and/or saccharification process, in which additional enzymatic activities, such as, alpha-amylase and glucoamylase activity are used in processing the substrate, e.g., a starch substrate.

In yet another preferred embodiment, the fermentation process is used in the production of ethanol. In accordance with this preferred embodiment, the application of the at least one esterase, which at least one esterase comprises a cutinase, can be used to raise the limit of ethanol tolerance of the fermenting microorganism to thereby improve ethanol yield and/or to increase the rate of fermentation. In a more preferred embodiment, at least one lipolytic enzyme is applied to raise ethanol tolerance of the fermenting microorganism to thereby improve ethanol yield.

In another preferred embodiment, additional enzyme activity or activities may be used in combination with (such as prior to, during or following) the esterase treatment of the present invention. Preferred additional enzymes include proteases, phytases, xylanases and maltogenic alpha-amylases.

### DETAILED DESCRIPTION OF THE INVENTION

"Fermentation" or "fermentation process" refers to any fermentation process or any process comprising a fermentation step. A fermentation process includes, without limitation, fermentation processes used to produce alcohols (e.g., ethanol, methanol, butanol); organic acids (e.g., citric acid, acetic acid, itaconic acid, lactic acid, gluconic acid); ketones (e.g., acetone); amino acids (e.g., glutamic acid); gases (e.g., H₂ and CO₂); antibiotics (e.g., penicillin and tetracycline); enzymes; vitamins (e.g., riboflavin, B₁₂, beta-carotene); and hormones. Fermentation processes also include fermentation processes used in the consumable alcohol industry (e.g., beer and wine), dairy industry (e.g., fermented dairy products), leather industry and tobacco industry. Preferred fermentation processes include alcohol fermentation processes, as are well known in the art. Preferred fermentation processes are anaerobic fermentation processes, as are well known in the art.

"Fermentation media" or "fermentation medium" refers to the environment in which the fermentation is carried out and which includes the fermentation substrate, that is, the carbohydrate source that is metabolized by the fermenting microorganism. The fermentation media, including fermentation substrate and other raw materials used in the fermentation process may be processed, e.g., by milling, liquefaction and saccharification processes or other desired processes prior to or simultaneously with the fermentation process. Accordingly, the fermentation media can refer to the media before the fermenting microorganisms are added, such as, the media in or resulting from a liquefaction or saccharification process, as well as the media which comprises the fermenting microorganisms, such as, the media used in a simultaneous saccharification and fermentation process (SSF).

The fermentation media or fermentation substrate may also comprise de-germed cereal grain, preferably de-germed corn, i.e. endosperm and torrified corn (e.g., heat-treated and poped corn) or any other cereal. The cereal grain can be ground or it can be fermented as whole.

"Fermenting microorganism" refers to any microorganism suitable for use in a desired fermentation process. Suitable fermenting microorganisms according to the invention are able to ferment, i.e., convert, sugars, such as glucose or maltose, directly or indirectly into the desired fermentation product. Examples of fermenting microorganisms include fungal organisms, such as yeast. Preferred yeast include strains of the *Sacchromyces* spp., and in particular, *Sacchromyces cerevisiae.* Commercially available yeast include, e.g., Red Star®/Lesaffre Ethanol Red (available from Red Star/Lesaffre, USA) FALI (available from Fleischmann's Yeast, a division of Burns Philp Food Inc., USA), SUPERSTART (available from Alltech), GERT STRAND (available from Gert Strand AB, Sweden) and FERMIOL (available from DSM Specialties).

An "esterase", also referred to as a carboxylic ester hydrolases, refers to enzymes acting on ester bonds, and includes enzymes classified in EC 3.1.1 Carboxylic Ester Hydrolases according to Enzyme Nomenclature (available at http://www.chem.qmw.ac.uk/iubmb/enzyme or from Enzyme Nomenclature 1992, Academic Press, San Diego, California, with Supplement 1 (1993), Supplement 2 (1994), Supplement 3 (1995), Supplement 4 (1997) and Supplement 5, in Eur. J. Biochem. 1994, 223, 1-5; Eur. J. Biochem. 1995, 232, 1-6; Eur. J. Biochem. 1996, 237, 1-5; Eur. J. Biochem. 1997, 250; 1-6, and Eur. J. Biochem. 1999, 264, 610-650; respectively). Non-limiting examples of esterases include arylesterase, triacylglycerol lipase, acetylesterase, acetylcholinesterase, cholinesterase, tropinesterase. pectinesterase, sterol esterase, chlorophyllase, L-arabinonolactonase, gluconolactonase, uronolactonase, tannase, retinyl-palmitate esterase, hydroxybutyrate-dimer hydrolase, acylglycerol lipase, 3-oxoadipate enol-lactonase, 1,4-lactonase, galactolipase, 4-pyridoxolactonase, acylcarnitine hydrolase, aminoacyl-tRNA hydrolase, D-arabinonolactonase, 6-phosphogluconolactonase, phospholipase A1, 6-acetylglucose deacetylase, lipoprotein lipase, dihydrocoumarin lipase, limonin-D-ring-lactonase, steroid-lactonase, triacetate-lactonase, actinomycin lactonase, orsellinate-depside hydrolase, cephalosporin-C deacetylase, chlorogenate hydrolase, alpha-amino-acid esterase, 4-methyloxaloacetate esterase, carboxymethylenebutenolidase, deoxylimonate A-ring-lactonase, 2-acetyl-1-alkylglycerophosphocholine esterase, fusarinine-C ornithinesterase, sinapine esterase, wax-ester hydrolase, phorbol-diester hydrolase, phosphatidylinositol deacylase, sialate O-acetylesterase, acetoxybutynylbithiophene deacetylase, acetylsalicylate deacetylase, methylumbelliferyl-acetate deacetylase, 2-pyrone-4,6-dicarboxylate lactonase, N-acetylgalactosaminoglycan deacetylase, juvenile-hormone esterase, bis(2-ethylhexyl)phthalate esterase, protein-glutamate methylesterase, 11-cis-retinyl-palmitate hydrolase, all-trans-retinyl-palmitate hydrolase, L-rhamnono-1,4-lactonase, 5-(3,4-diacetoxybut-1-ynyl)-2,2'-bithiophene deacetylase, fatty-acyl-ethyl-ester synthase, xylono-1,4-lactonase, N-acetylglucosaminylphosphatidylinositol deacetylase, cetraxate benzylesterase, acetylalkylglycerol acetylhydrolase, and acetylxylan esterase.
The cutinases (as classified by EC 3.1.1.74) used in the present invention are esterases. Other esterases for use in the present invention in combination with cutinases are lipolytic enzymes, such as, lipases (as classified by EC 3.1.1.3, EC 3.1.1.23 and/or EC 3.1.1.26) and phospholipases (as classified by EC 3.1.1.4 and/or EC 3.1.1.32, including lysophospholipases (as classified by EC 3.1.1.5).

The present invention provides methods and compositions for producing a fermentation product in which at least one esterase enzyme is used in the fermentation process, wherein said at least one esterase enzyme comprises a cutinase. The esterase treatment may be applied at any stage in a fermentation process. In a preferred embodiment, the esterase is added in an effective amount during fermentation (such as, by contacting the fermentation medium), such as, at the start of the fermentation process. In another preferred embodiment, the esterase is added in an effective amount prior to fermentation, such as, during propogation of the fermenting organisms or during liquefaction, saccharification or a pre-saccharification step. The addition of an esterase in a fermentation process, such as, in an ethanol production process, for example, can be used to raise the limit of ethanol tolerance of the fermenting microorganism and to thereby improve yield of the fermentation product (ethanol yield) by converting components in the fermentation medium, such as, oil from the corn substrate, to components useful the fermenting microorganism, e.g., unsaturated fatty acids and glycerol.

Any suitable substrate or raw material may be used in the fermentation processes of the present invention. The substrate is generally selected based on the desired fermentation product and the process employed, as is well known in the art. Examples of substrates suitable for use in the processes of present invention, include starch-containing materials, such as tubers, roots, whole grains, corns, cobs, wheat, barley, rye, milo or cereals, sugar-containing raw materials, such as molasses, fruit materials, sugar, cane or sugar beet, potatoes, and cellulose-containing materials, such as wood or plant residues. Suitable substrates also include carbohydrate sources, in particular, low molecular sugars DP₁₋₃ that can be metabolized by the fermenting microorganism, and which may be supplied by direct addition to the fermentation media.

The esterase will be added in an amount effective to improve fermentation, e.g., to change the lipid composition/concentration inside and/or outside of the fermenting microorganism or in the cell membrane of the fermenting microorganism, to result in an improvement in the movement of solutes into and/or out of the fermenting microorganisms during fermentation and/or to provide more metabolizable energy sources (such as, e.g., by converting components, such as, oil from the corn substrate, to components useful the fermenting microorganism, e.g., unsaturated fatty acids and glycerol), to increase ethanol yield. Examples of effective amounts of esterase include 0.5 to 1000 U/g DS% (dry solid percentage) in fermentation media, preferably 1 to 400 U/g %DS, and more preferably 1 to 20 U/g DS%, such as, 1 to 5 U/g DS%. Further optimization of the amount of esterase can hereafter be obtained using standard procedures known in the art.

The conditions for the esterase treatment (e.g., pH, temperature and treatment time), will depend on the application (e.g., applying the esterase treatment to a raw starch hydrolysis process as compared to applying the esterase in a traditional starch hydrolysis process in which the starch substrate is gelatinized). Factors to consider when selecting an esterase should therefore include the conditions which will be employed during the treatment. For example, raw starch hydrolysis processes are preferably carried out at 32.2 °C for 64h, with the initial pH of about 4.5-5.0. In some cases, temperature staging can be used to lower the stress impact on yeast. Time of fermentation can vary depending on ethanol yield to be expected.

The esterase treatment will also be a function of the yeast used in fermentation, in particular, the yeast genetics and yeast physiology. Accordingly, the optimum conditions (e.g., pH, temperature, treatment time, esterase selection and concentration) for the esterase treatment may vary depending on the yeast used. Accordingly, the esterase treatment employed, e.g., staging conditions, esterase concentration, and esterase selection, can hereafter be optimized for the yeast used in the fermentation process. Preferably, the esterase treatment is carried out under high ethanol concentration conditions, i.e., concentration of up to 22% v/v.

In the present invention, the at least one esterase comprises a cutinase. Cutinases are enzymes which are able to degrade cutin. The cutinase may be derived from any source. In a preferred embodiment, the cutinase is derived from a strain of *Aspergillus,* in particular *Aspergillus oryzae,* a strain of *Alternaria,* in particular *Alternaria brassiciola,* a strain of Fusarium, in particular *Fusarium solani, Fusarium solani pisi, Fusarium roseum culmorum,* or *Fusarium roseum sambucium,* a strain of *Helminthosporum,* in particular *Helminthosporum sativum,* a strain of *Humicola,* in particular *Humicola insolens,* a strain of *Pseudomonas,* in particular *Pseudomonas mendocina,* or *Pseudomonas putida,* a strain of *Rhizoctonia,* in particular *Rhizoctonia solani,* a strain of *Streptomyces,* in particular *Streptomyces scabies,* or a strain of *Ulocladium,* in particular *Ulocladium consortiale.* In a most preferred embodiment the cutinase is derived from a strain of *Humicola insolens,* in particular the strain *Humicola insolens* DSM 1800. Humicola insolens *cutinase* is described in WO 96/13580, which is herby incorporated by reference. Preferred cutinases include JC 492 (available from Novozymes A/S).

Cutinases are preferably added in amounts from about 0.5 to 1000 U/g DS% in fermentation media, preferably, 1 to 400 U/g DS%, such as, 40 to 400 U/g DS%, more preferably, 1 to 20 U/g DS%, such as, 1 to 10 U/g DS% and 1 to 5 U/g DS%.

In a preferred embodiment of the present invention, the at least one esterase furthermore comprises a lipolytic enzyme, more preferably, a lipase. As used herein, a "lipolytic enzymes" refers to lipases and phospholipases (including lyso-phospholipases). The lipolytic enzyme is preferably of microbial origin, in particular of bacterial, fungal or yeast origin. The lipolytic enzyme used may be derived from any source, including, for example, a strain of *Absidia,* in particular *Absidia blakesleena* and *Absidia corymbifera,* a strain of *Achromobacter,* in particular *Achromobacter iophagus,* a strain of *Aeromonas,* a strain of *Alternaria,* in particular *Alternaria brassiciola, a* strain of *Aspergillus,* in particular *Aspergillus niger* and *Aspergillus flavus,* a strain of *Achromobacter,* in particular *Achromobacter iophagus,* a strain of *Aureobasidium,* in particular Aureo*basidium pullulans,* a strain of *Bacillus,* in particular *Bacillus pumilus, Bacillus strearothermophilus* and *Bacillus subtilis,* a strain of *Beauveria,* a strain of *Brochothrix,* in particular *Brochothrix thermosohata,* a strain of *Candida,* in particular *Candida cylindracea (Candida rugosa), Candida paralipolytica,* and *Candida antarctica,* a strain of *Chromobacter,* in particular *Chromobacter viscosum,* a strain of *Coprinus,* in particular *Coprinus cinerius,* a strain of *Fusarium,* in particular *Fusarium oxysporum, Fusarium solani, Fusarium solani pisi,* and *Fusarium roseum culmorum,* a strain of *Geotricum,* in particular *Geotricum penicillatum,* a strain of *Hansenula,* in particular *Hansenula anomala,* a strain of *Humicola,* in particular *Humicola brevispora, Humicola brevis* var. thermoidea, and *Humicola insolens,* a strain of *Hyphozyma,* a strain of *Lactobacillus,* in particular *Lactobacillus curvatus,* a strain of *Metarhizium,* a strain of *Mucor,* a strain of *Paecilomyces,* a strain of *Penicillium,* in particular *Penicillium cyclopium, Penicillium crustosum* and *Penicillium expansum,* a strain of *Pseudomonas* in particular *Pseudomonas aeruginosa, Pseudomonas alcaligenes, Pseudomonas cepacia* (syn. *Burkholderia cepacia), Pseudomonas fluorescens, Pseudomonas fragi, Pseudomonas maltophilia, Pseudomonas mendocina, Pseudomonas mephitica lipolytica, Pseudomonas alcaligenes, Pseudomonas plantari, Pseudomonas pseudoalcaligenes, Pseudomonas putida, Pseudomonas stutzeri,* and *Pseudomonas wisconsinensis,* a strain of *Rhizoctonia,* in particular *Rhizoctonia solani,* a strain of *Rhizomucor,* in particular *Rhizomucor miehei,* a strain of *Rhizopus,* in particular *Rhizopus japonicus, Rhizopus microsporus* and *Rhizopus nodosus,* a strain of *Rhodosporidium,* in particular *Rhodosporidium toruloides,* a strain of *Rhodotorula,* in particular *Rhodotorula glutinis,* a strain of *Sporobolomyces,* in particular *Sporobolomyces shibatanus,* a strain of *Thermomyces,* in particular *Thermomyces lanuginosus* (formerly *Humicola lanuginosa),* a strain of *Thiarosporella,* in particular *Thiarosporella phaseolina,* a strain of *Trichoderma,* in particular *Trichoderma harzianum,* and *Trichoderma reesei,* and/or a strain of *Verticillium.*

In a preferred embodiment, the lipolytic enzyme used according to the invention is derived from a strain of *Aspergillus,* a strain of *Achromobacter,* a strain of *Bacillus,* a strain of *Candida, a* strain of *Chromobacter,* a strain of *Fusarium,* a strain of *Humicola,* a strain of *Hyphozyma, a* strain of *Pseudomonas,* a strain of *Rhizomucor,* a strain of *Rhizopus,* or a strain of *Thermomy*ces.

In more preferred embodiments, the lipolytic enzymes is a lipase. Lipases may be applied herein, e.g., for their ability to modify the structure and composition of triglyceride oils and fats in the fermentation media (including fermentation yeast), for example, resulting from a corn substrate. Lipases catalyze different types of triglyceride conversions, such as hydrolysis, esterification and transesterification. Suitable lipases include acidic, neutral and basic lipases, as are well-known in the art, although acidic lipases (such as, e.g., the lipase G AMANO 50, available from Amano) appear to be more effective at lower concentrations of lipase as compared to either neutral or basic lipases. Preferred lipases for use in the present invention included *Candida antarcitca* lipase and *Candida cylindracea* lipase. More preferred lipases are purified lipases such as *Candida antarcitca* lipase (lipase A), *Candida antarcitca* lipase (lipase B), *Candida cylindracea* lipase, and *Penicillium camembertii* lipase.

Preferred commercial lipases include LIPOLASE and LIPEX (available from Novozymes A/S) and G AMANO 50 (available from Amano).

Lipases are preferably added in amounts from about 0.5 to 1000 LU/g DS% in fermentation media, preferably, 1 to 400 LU/g DS, more preferably 1 to 20 LU/g DS%, such as, 1 to 10 LU/g DS% and 1 to 5 LU/g DS%.

In another preferred embodiment, the at least one esterase, which at least one esterase enzyme comprises a cutinase furthermore comprises a phospholipase. Phospholipases are enzymes which have activity towards phospholipids. Phospholipids, such as lecithin or phosphatidylcholine, consist of glycerol esterified with two fatty acids in an outer (sn-1) and the middle (sn-2) positions and esterified with phosphoric acid in the third position; the phosphoric acid, in turn, may be esterified to an amino-alcohol. Phospholipases are enzymes which participate in the hydrolysis of phospholipids. Several types of phospholipase activity can be distinguished, including phospholipases A₁ and A₂ which hydrolyze one fatty acyl group (in the sn-1 and sn-2 position, respectively) to form lysophospholipid; and lysophospholipase (or phospholipase B) which can hydrolyze the remaining fatty acyl group in lysophospholipid. Phospholipase C and phospholipase D (phosphodiesterases) release diacyl glycerol or phosphatidic acid respectively.

The term phospholipase includes enzymes with phospholipase activity, e.g. phospholipase A (A₁ or A₂), phospholipase B activity, phospholipase C activity or phospholipase D activity. The term "phospholipase A" used herein in connection with an enzyme of the invention is intended to cover an enzyme with Phospholipase A₁ and/or Phospholipase A₂ activity. The phospholipase activity may be provided by enzymes having other activities as well, such as, e.g., a lipase with phospholipase activity. The phospholipase activity may, e.g., be from a lipase with phospholipase side activity. In other embodiments of the invention the phospholipase enzyme activity is provided by an enzyme having essentially only phospholipase activity and wherein the phospholipase enzyme activity is not a side activity.

The phospholipase may be of any origin, e.g. of animal origin (such as, e.g. mammalian), e.g. from pancreas (e.g. bovine or porcine pancreas), or snake venom or bee venom. Alternatively, the phospholipase may be of microbial origin, e.g. from filamentous fungi, yeast or bacteria, such as the genus or species *Aspergillus,* e.g. *A. niger, Dictyostelium,* e.g. *D. discoideum; Mucor,* e.g. *M. javanicus, M. mucedo, M. subtilissimus; Neurospora,* e.g. *N*. *crassa; Rhizomucor,* e.g. *R. pusillus; Rhizopus,* e.g. *R*. *arrhizus, R. japonicus, R. stolonifer, Sclerotinia,* e.g. *S. libertiana; Trichophyton,* e.g. *T. rubrum; Whetzelinia,* e.g. *W. sclerotiorum; Bacillus,* e.g. *B. megaterium, B. subtilis; Citrobacter,* e.g. *C*. *freundii; Enterobacter,* e.g. *E. aerogenes, E. cloacae Edwardsiella, E. tarda; Erwinia,* e.g. *E*. *herbicola; Escherichia,* e.g. *E. coli; Klebsiella,* e.g. *K. pneumoniae; Proteus,* e.g. *P. vulgaris; Providencia,* e.g. *P. stuartii; Salmonella,* e.g. *S*. *typhimurium; Serratia,* e.g. *S. liquefasciens, S. marcescens; Shigella,* e.g. *S. flexneri; Streptomyces,* e.g. *S. violeceoruber, Yersinia,* e.g. *Y. enterocolitica.* Thus, the phospholipase may be fungal, e.g. from the class *Pyrenomycetes,* such as the genus *Fusarium,* such as a strain of *F. culmorum, F. heterosporum, F. solani,* or a strain of *F. oxysporum.* The phospholipase may also be from a filamentous fungus strain within the genus *Aspergillus,* such as a strain of *Aspergillus awamori, Aspergillus foetidus, Aspergillus japonicus, Aspergillus niger* or *Aspergillus oryzae.* Preferred commercial phospholipases include LECITASE and LECITASE ULTRA (available from Novozymes A/S).

Phospholipases are preferably added in amounts from about 0.5 to 1000 PLA/U/g DS% in fermentation media, preferably, 1 to 400 PLA/U/g DS%, more preferably, 1 to 20 PLA/U/g DS%, such as, 1-10 PLA/U/g DS%.

In another preferred embodiment, combinations of esterases are used, such as (1) a lipase and a cutinase; (2) a lipase and a phospholipase and a cutinase; and (3) a phospholipase and a cutinase.

In another preferred embodiment, additional enzyme activity or activities may be used in combination with (such as prior to, during or following) the esterase treatment of the present invention. In addition to combining esterase with the enzymes traditionally used in starch processing, e.g., alpha-amylases and glucoamylases, preferred additional enzymes also include proteases, phytases, xylanases, cellulases, maltogenic alpha-amylases and beta-amylases. More preferably, the additional enzymes are proteases, phytases, xylanases, maltogenic alpha-amlylases and beta-amylases.

In a preferred embodiment, the esterase treatment is used in combination with a phytase. In accordance with this embodiment, a phytase may be used, e.g., to promote the liberation of inorganic phosphate from phytic acid (myo-inositol hexakisphosphate) or from any salt thereof (phytates) present in the medium. The phytase may be added during the fermentation or prior to fermentation, such as, during propogation or in a step prior to fermentation, e.g., a liquefaction and/or saccharification step. The phytases made by added, e.g., to improve the bioavailability of essential minerals to yeast, as described in PCT Application WO 01/62947, which is hereby incorporated by reference.

A "phytase" is an enzyme which is able to effect the liberation of inorganic phosphate from phytic acid (myo-inositol hexakisphosphate) or from any salt thereof (phytates). Phytases can be classified according to their specificity in the initial hydrolysis step, viz. according to which phosphate-ester group is hydrolyzed first. The phytase to be used in the invention may have any specificity, e.g., a 3-phytase (E.C. 3.1.3.8), a 6-phytase (E.C. 3.1.3.26) or a 5-phytase (no E.C. number).

The phytase may be derived from plants or microorganisms, such as bacteria or fungi, e.g., yeast or filamentous fungi. The plant phytase may be from wheat-bran, maize, soy bean or lily pollen. Suitable plant phytases are described in Thomlinson et al, Biochemistry, 1 (1962), 166-171; Barrientos et al, Plant. Physiol., 106 (1994), 1489-1495; WO 98/05785; WO 98/20139.
A bacterial phytase may be from genus *Bacillus, Pseudomonas* or *Escherichia,* preferably the species *B. subtilis* or *E. coli.* Suitable bacterial phytases are described in Paver and Jagannathan, 1982, Journal of Bacteriology 151:1102-1108; Cosgrove, 1970, Australian Journal of Biological Sciences 23:1207-1220; Greiner et al, Arch. Biochem. Biophys., 303, 107-113,1993; WO 98/06856; WO 97/33976; WO 97/48812.

A yeast phytase or myo-inositol monophosphatase may be derived from genus *Saccharomyces* or *Schwanniomyces,* preferably species *Saccharomyces cerevisiae* or *Schwanniomyces occidentalis.* Suitable yeast phytases are described in Nayini et al, 1984, Lebensmittel Wissenschaft und Technologie 17:24-26; Wodzinski et al, Adv. Appl. Microbiol., 42, 263-303; AU-A-24840/95;

Phytases from filamentous fungi may be derived from the fungal phylum of *Ascomycota* (ascomycetes) or the phylum *Basidiomycota,* e.g., the genus *Aspergillus, Thermomyces* (also called *Humicola), Myceliophthora, Manascus, Penicillium, Peniophora, Agrocybe, Paxillus,* or *Trametes,* preferably the species *Aspergillus terreus, Aspergillus niger, Aspergillus niger var. awamori, Aspergillus ficuum, Aspergillus fumigatus, Aspergillus oryzae, T. lanuginosus* (also known as *H. lanuginosa), Myceliophthora thermophila, Peniophora lycii, Agrocybe pediades, Manascus anka, Paxillus involtus,* or *Trametes pubescens.* Suitable fungal phytases are described in Yamada et al., 1986, Agric. Biol. Chem. 322:1275-1282; Piddington et al., 1993, Gene 133:55-62; EP 684,313; EP 0 420 358; EP 0 684 313; WO 98/28408; WO 98/28409; JP 7-67635; WO 98/44125; WO 97/38096; WO 98/13480.

Modified phytases or phytase variants are obtainable by methods known in the art, in particular by the methods disclosed in EP 897010; EP 897985; WO 99/49022; WO 99/48330. Commercially available phytases include BIO-FEED PHYTASE^{™}, PHYTASE NOVO^{™} CT or L (Novozymes A/S), or NATUPHOS^{™} NG 5000 (DSM).

The phytase may preferably be added in an amount of .005 to 250 FYT/g DS%, preferably 10 to 100 FYT/g DS%. A preferred suitable dosage of the phytase is in an amount of 0.005-25 FYT/g DS%, more preferably 0.01-10 FYT/g, such as 0.1-1 FYT/g DS%. Here, the phytase activity is determined using FYT units, one FYT being the amount of enzyme that liberates 1 micromole inorganic ortho-phosphate per min. under the following conditions: pH 5.5; temperature 37°C; substrate: sodium phytate (C₆H₆O₂₄P₆Na₁₂) at a concentration of 0.0050 mole/l.

In another preferred embodiment, the esterase treatment is used in combination with a protease. The protease may be used, e.g., to digest protein to produce free amino nitrogen (FAN). Such free amino acids function as nutrients for the yeast, thereby enhancing the growth of the yeast and, consequently, the production of ethanol.

Furthermore, although increased glycerol concentration resulting from the esterase treatment can raise the ethanol tolerance of the yeast and improve ethanol yield, a glycerol concentration which is too high can have a detrimental effect on the performance of yeast. In the event that this occurs, the protease treatment may accordingly also be used to reduce or maintain the glycerol concentration within the desired limits preferable to the fermentation microorganism.

Proteases are well known in the art and refer to enzymes that catalyze the cleavage of peptide bonds. Suitable proteases include fungal and bacterial proteases. Preferred proteases are acidic proteases, i.e., proteases characterized by the ability to hydrolyze proteins under acidic conditions below pH 7. Suitable acid fungal proteases include fungal proteases derived from *Aspergillus, Mucor, Rhizopus, Candida, Coriolus, Endothia, Enthomophtra, Irpex, Penicillium, Sclerotium* and *Torulopsis.* Especially contemplated are proteases derived from *Aspergillus niger (see, e.g.,* Koaze et al., (1964), Agr. Biol. Chem. Japan, 28, 216*), Aspergillus saitoi (see, e.g.,* Yoshida, (1954) J. Agr. Chem. Soc. Japan, 28, 66*), Aspergillus awamori* (Hayashida et al., (1977) Agric. Biol. Chem., 42(5), 927-933, *Aspergillus aculeatus* (WO 95/02044), or *Aspergillus oryzae;* and acidic proteases from *Mucor pusillus* or *Mucor miehei.*

Commercial proteases include GC 106 and SPEZYME FAN (available from Genencor). Suitable bacterial proteases, although not acidic proteases, include the commercially available products Alcalase® and Neutrase® (available from Novozymes A/S).

Preferably, the protease is an aspartic acid protease, as described, for example, Handbook of Proteolytic Enzymes, Edited by A.J. Barrett, N.D. Rawlings and J.F. Woessner, Academic Press, San Diego, 1998, Chapter 270). Suitable examples of aspartic acid protease include, e.g., those disclosed in R.M. Berka et al. Gene, 96, 313 (1990)); (R.M. Berka et al. Gene, 125, 195-198 (1993)); and Gomi et al. Biosci. Biotech. Biochem. 57, 1095-1100 (1993), which are hereby incorporated by reference.

Protease may preferably be added in an amount of an amount of 10⁻⁷ to 10⁻⁵ gram active protease protein/g DS%, in particular 10⁻⁷ to 5x10⁻⁶ gram active protease protein/g DS%

In yet another preferred embodiment, the esterase treatment is used in combination with a maltogenic alpha-amylase. A "maltogenic alpha-amylase" (glucan 1,4-α-maltohydrolase, E.C. 3.2.1.133) is able to hydrolyze amylose and amylopectin to maltose in the alpha-configuration. Examples of maltogenic alpha-amylases include the maltogenic alpha-amylase from *B*. *stearothermophilus* strain NCIB 11837. Maltogenic alpha-amylases are described in US Patent nos. 4,598,048, 4,604,355 and 6,162,628, which are hereby incorporated by reference. A commercially available maltogenic amylase is MALTOGENASE^{™} (available from Novozymes A/S). Preferably, the maltogenic alpha-amylase is used in a raw starch hydrolysis process to aid the formation of retrograded starch. Preferably, the esterase is combined with the maltogenic alpha-amylase in a liquefaction process. Preferably, the maltogenic alpha-amylase is added in an amount of 0.02 to 1.0 g/DS%.

In yet another preferred embodiment, the esterase treatment is used in combination with a beta-amylase. Beta-amylase (E.C 3.2.1.2) is the name traditionally given to exo-acting maltogenic amylases, which catalyze the hydrolysis of 1,4-alpha-glucosidic linkages in amylose, amylopectin and related glucose polymers. Maltose units are successively removed from the non-reducing chain ends in a step-wise manner until the molecule is degraded or, in the case of amylopectin, until a branch point is reached. The maltose released has the beta anomeric configuration, hence the name beta-amylase.

Beta-amylases have been isolated from various plants and microorganisms (W.M. Fogarty and C.T. Kelly, Progress in Industrial Microbiology, vol. 15, pp. 112-115, 1979). These beta-amylases are characterized by having optimum temperatures in the range from 40°C to 65°C and optimum pH in the range from 4.5 to 7. Other examples of beta-amylase include the beta-amylases described in U.S. Patent No. 5,688,684. Commercially available beta-amylases include NOVOZYM WBA (from Novozymes A/S) and SPEZYME^{™} BBA 1500 and OPTIMALT (from Genencor Int., USA).

In another preferred embodiment, the esterase treatment is used in combination with an xylanase. The xylanase (E.C. 3.2.1.8) activity may be derived from any suitable source, including fungal and bacterial organisms, such as *Aspergillus, Disporotrichum, Penicillium, Neurospora, Fusarium* and *Trichoderma.* Preferred commercially available preparations comprising xylanase include SHEARZYME®, BIOFEED WHEAT®, CELLUCLAST®, ULTRAFLO®, VIS-COZYME® (Novozymes A/S) and SPEZYME® CP (Genencor Int.).

In yet another preferred, the esterase treatment is used in combination with a cellulase. The cellulase activity used according to the invention may be derived from any suitable origin, preferably, the cellulase is of microbial origin, such as derivable from a strain of a filamentous fungus (e.g., *Aspergillus, Trichoderma, Humicola, Fusarium*). Commercially available preparations comprising cellulase which may be used include CELLUCLAST®, CEL-LUZYME®, CEREFLO® and ULTRAFLO® (Novozymes A/S), LAMINEX^{™} and SPEZYME® CP (Genencor Int.) and ROHAMENT® 7069 W (from Röhm GmbH).

The fermentation processes described herein are preferably used in combination with liquefaction or saccharification processes. Any liquefaction or saccharification may be used in combination with the fermentation process of the present invention. According to the present invention, the saccharification and liquefaction may be carried out simultaneously or separately with the fermentation process. In a preferred embodiment of the present invention, the liquefaction, saccharification and fermentation processes are carried out simultaneously.

"Liquefaction" is a process in which milled (whole) grain raw material is broken down (hydrolyzed) into maltodextrins (dextrins). Liquefaction is often carried out as a three-step hot slurry process. The slurry is heated to between 60-95°C, preferably 80-85°C, and the enzymes are added to initiate liquefaction (thinning). The slurry is then jet-cooked at a temperature between 95-140°C, preferably 105-125°C to complete gelatinization of the slurry. Then the slurry is cooled to 60-95°C and more enzyme(s) is(are) added to finalize hydrolysis (secondary liquefaction). The liquefaction process is usually carried out at pH 4.5-6.5, in particular at a pH between 5 and 6. Milled and liquefied whole grains are known as mash.

The liquefaction processes are typically carried out using an alpha-amylase. Preferred alpha-amylases are of fungal or bacterial origin. More preferably, the alpha-amylase is a *Bacillus* alpha-amylases, such as, derived from a strain of *B. licheniformis, B. amyloliquefaciens,* and *B. stearothermophilus.* Other alpha-amylases include alpha-amylase derived from a strain of the *Bacillus* sp. NCIB 12289, NCIB 12512, NCIB 12513 or DSM 9375, all of which are described in detail in WO 95/26397, and the alpha-amylase described by Tsukamoto et al., Biochemical and Biophysical Research Communications, 151 (1988), pp. 25-31. Other alpha-amylase variants and hybrids are described in WO 96/23874, WO 97/41213, and WO 99/19467. Other alpha-amylase include alpha-amylases derived from a strain of *Aspergillus,* such as, *Aspergillus oryzae* and *Aspergillus niger* alpha-amylases.

In a preferred embodiment, the alpha amylase is an acid alpha amylases. The term "acid alpha-amylase" means an alpha-amylase (E.C. 3.2.1.1) which when added in an effective amount has activity at a pH in the range of 3.0 to 7.0, preferably from 3.5 to 6.0, or more preferably from 4.0-5.0. Any suitable acid alpha-amylase may be used in the present invention.

In a preferred embodiment, the acid alpha-amylase is an acid fungal alpha-amylase or an acid bacterial alpha-amylase. Preferred acid alpha-amylase for use in the present invention may be derived from a strain of *B. licheniformis, B. amyloliquefaciens,* and *B. stearothermophilus.* More preferably, the acid alpha-amylase is an acid fungal alpha amylases, such as, SP 288 (available from Novozymes).

Preferred commercial compositions comprising alpha-amylase include MYCOLASE (Gist Brocades), BAN^{™}, TERMAMYL^{™} SC, FUNGAMYL^{™}, LIQUOZYME^{™} X and SAN^{™} SUPER, SAN^{™} EXTRA L (Novozymes A/S) and CLARASE L-40,000, DEX-LO^{™}, SPEYME FRED, SPEZYME^{™} AA, and SPEZYME^{™} DELTA AA (Genencor Int.).

The alpha-amylase may be added in amounts as are well-known in the art. When measured in AAU units the acid alpha-amylase activity is preferably present in an amount of 5-500000 AAU/kg of DS, in an amount of 500-50000 AAU/kg of DS, or more preferably in an amount of 100-10000 AAU/kg of DS, such as 500-1000 AAU/kg DS. Fungal acid alpha-amylase are preferably added in an amount of 10-10000 AFAU/kg of DS, in an amount of 500-2500 AFAU/kg of DS, or more preferably in an amount of 100-1000 AFAU/kg of DS, such as approximately 500 AFAU/kg DS.

"Saccharification" is a process in which the maltodextrin (such as, produced from the liquefaction process) is converted to low molecular sugars DP₁₋₃ (i.e., carbohydrate source) that can be metabolized by the fermenting organism, such as, yeast. Saccharification processes are well known in the art and are typically performed enzymatically using a glucoamylase. Alternatively or in addition, alpha-glucosidases or acid alpha-amylases may be used. A full saccharification process may last up to from about 24 to about 72 hours, and is often carried out at temperatures from about 30 to 65 degrees Celsius, and at a pH between 4 and 5, normally at about pH 4.5. However, it is often more preferred to do a pre-saccharification step, lasting for about 40 to 90 minutes, at temperature of between 30-65°C, typically about 60°C, followed by complete saccharification during fermentation in a simultaneous saccharification and fermentation process (SSF).

The glucoamylase used in the saccharification process may be derived from any suitable source, e.g., derived from a microorganism or a plant. Preferred glucoamylases are of fungal or bacterial origin, selected from the group consisting of *Aspergillus* glucoamylases, in particular *A. niger* G1 or G2 glucoamylase (Boel et al. (1984), EMBO J. 3 (5), p. 1097-1102), or variants thereof, such as disclosed in WO 92/00381 and WO 00/04136; the *A. awamori* glucoamylase (WO 84/02921), A. oryzae (Agric. Biol. Chem. (1991), 55 (4), p. 941-949), or variants or fragments thereof.

Other *Aspergillus* glucoamylase variants include variants to enhance the thermal stability, such as, G137A and G139A (Chen et al. (1996), Prot. Engng. 9,499-505); D257E and D293E/Q (Chen et al. (1995), Prot. Engng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Engng. 10, 1199-1204. Other glucoamylases include *Talaromyces* glucoamylases, in particular, derived from *Talaromyces emersonii* (WO 99/28448), *Talaromyces leycettanus* (US patent no. Re. 32,153), *Talaromyces duponti, Talaromyces thermophilus* (US patent no. 4,587,215). Bacterial glucoamylases contemplated include glucoamylases from the genus *Clostridium,* in particular C. *thermoamylolyticum (*EP 135,138), and C. *thermohydrosulfuricum* (WO 86/01831).

Commercially available compositions comprising glucoamylase include AMG 200L; AMG 300 L; SAN^{™} SUPER, SAN EXTRA L, SPIRIZYME PLUS and AMG^{™} E (from Novozymes A/S); AMIGASE^{™} and AMIGASE^{™} PLUS (from DSM); OPTIDEX^{™} 300, G-ZYME^{™} G900, G-ZYME^{™} and G990 ZR (from Genencor Int.).

Glucoamylases may in an embodiment be added in an amount of 0.02-2 AGU/g DS, preferably 0.1-1 AGU/g DS, such as 0.2 AGU/g DS.

The most widely used process in ethanol production is the simultaneous saccharification and fermentation (SSF) process, in which there is no holding stage for the saccharification, meaning that fermenting organism, such as the yeast, and enzyme(s) is(are) added together. In SSF processes, it is common to introduce a pre-saccharification step at a temperature above 50°C, just prior to the fermentation.

More preferably, the liquefaction, saccharification or fermentation process is a simultaneous liquefaction-saccharification-fermentation (LSF) process also referred as a single enzymatic process, in which the liquefaction, saccharification and fermentation process are all carried out in one process, that is, all enzymes (or substitutable or additional non-enzymatic agents) and the yeast used for liquefaction, saccharification and fermentation, accordingly, are added in the same process step, more preferably, simultaneously in the same process step.

Preferred process conditions for LSF process include temperatures of about 26°C to 40°C, preferably about 32°C, pH of about 4 to about 8, preferably about pH 5, and process times of about 48 to 72 hours, preferably about 72 hours.

Preferably, the LSF process or single enzymatic process is a raw starch hydrolysis (RSH) processes, more preferably, used in the production of alcohol, such as, e.g., ethanol. A "raw starch hydrolysis" process (RSH) differs from conventional starch treatment processes in that raw uncooked starch, also referred to as granular starch, is used in the ethanol fermentation process. As used herein, the term "granular starch" means raw uncooked starch, i.e. starch in its natural form found, e.g., in cereal, tubers or grains. Starch is formed within plant cells as tiny granules insoluble in water. When put in cold water, the starch granules may absorb a small amount of the liquid and swell. At temperatures up to 50°C to 75°C the swelling may be reversible. However, with higher temperatures an irreversible swelling called gelatinization begins.

The term "initial gelatinization temperature" means the lowest temperature at which gelatinization of the starch commences. Starch heated in water begins to gelatinize between 50°C and 75°C; the exact temperature of gelatinization depends on the specific starch, and can readily be determined by the skilled artisan. For example, the initial gelatinization temperature may vary according to the plant species, to the particular variety of the plant species as well as with the growth conditions. In the context of this invention the initial gelatinization temperature of a given starch is the temperature at which birefringence is lost in 5% of the starch granules using the method described by Gorinstein. S. and Lii. C., Starch/Stärke, Vol. 44 (12) pp. 461-466 (1992).

In accordance with a preferred embodiment, esterases can be used, preferably in combination with an amylase and/or a glucoamylase, to increase ethanol yield in raw starch hydrolysis processes. Although not limited to any one theory of operation, it is believed that the esterase treatment improves the raw starch hydrolysis processes by one or more of the following mechanisms:
a) Supplementation of Yeast with Esterase-Generated Exogenous Unsaturated Fatty Acids. For example, in corn fermentation, esterases such as lipases can stimulate both yeast growth and performance by converting corn oil into unsaturated fatty acids which are useful to the yeast.
b) Inhibition of Ester Synthesis by Esterase-Generated Exogenous Unsaturated Fatty Acids. There is an additional advantage of supplying yeast with exogenous unsaturated fatty acids in that the unsaturated fatty acids can increase ethanol yield suppressing ethyl ester synthesis due to the inhibition of yeast alcohol acetyltransferase by exogenous unsaturated fatty acids. Moreover, fatty acids, such as corn-derived fatty acids produced in a raw starch hydrolysis process can be very valuable by-products. Linoleic acid, for example, is a component of vitamin F and is in a category of essential fatty acids, and alpha-linoleic acid is converted by human body into omega-3 fatty acids, which perform a number of important regulatory functions.
c) Promoting Starch Release. In raw starch hydrolysis, esterases, such as, lipases and phospholipases, can be used to facilitate starch release by acting on amyloplast membranes and starch-lipid complexes.
d) Glycerol Formation/Concentration. Production of ethanol from glucose is redox neutral process while glycerol formation maintains the redox balance of the yeast at the expense of carbohydrate conversion to ethanol. Most of glycerol is produced during the early stages of fermentation as a result of yeast growth when nearly all NADH is formed in de *novo* amino acid synthesis from ammonia and glucose. The esterase treatment can be used to increase glycerol concentration, which has a beneficial effect on the yeast's ethanol tolerance. However, if glycerol concentration becomes too high, a protease or other suitable glycerol reducing agent can be added to reduce the glycerol concentration.
e) Free fatty acids may function as foam breakers. Presence of free fatty acids relates to foam instability, and the esterase treatment can also reduce foam formation during the fermentation process.

In a preferred embodiment, the present invention involves treating granular starch slurry with a glucoamylase and/or alpha-amylase, yeast and at least one esterase at a temperature below the initial gelatinization temperature of granular starch. Preferably, the yeast is Ethanol Red yeast. The amylase is preferably an acid alpha-amylase, more preferably an acid fungal alpha-amylase, such as, SP 288 (from Novozymes).

In a more preferred embodiment, the raw starch hydrolysis process entails, treating granular starch slurry with a glucoamylase and/or alpha-amylase at a temperature between 0°C and 20°C below the initial gelatinization temperature of the granular starch, followed by treating the slurry with a glucoamylase and/or alpha amylase, yeast and at least one esterase at a temperature of between 10°C and 35°C.

In yet another preferred embodiment, the process entails the sequential steps of: (a) treating a granular starch slurry with an acid alpha-amylase and a glucoamylase at a temperature of 0°C to 20°C below the initial gelatinization temperature of the granular starch, preferably for a period of 5 minutes to 12 hours, (b) treating the slurry in the presence of an acid alpha-amylase, a glucoamylase, a yeast and at least one esterase at a temperature of between 10°C and 35°C, preferably for a period of 20 to 250 hours to produce ethanol.

Other enzymes and fermentation stimulators may be used in combination with the esterase treatment in the RSH process. Preferably, the other enzyme is selected from the group consisting of a phytase, protease, xylanase, cellulase, a maltogenic alpha-amylase and combinations thereof. In RSH processes, phytic acid is present in significant amounts. Accordingly, in a preferred embodiment, phytases can be used to promote the liberation of inorganic phosphate from phytic acid (myo-inositol hexakisphosphate) or from any salt thereof (phytates), as previously described.

In another preferred embodiment, a maltogenic alpha-amylase is used in combination with the esterase treatment in the RSH process.

A preferred application of the fermentation processes and compositions described herein is in an alcohol production process (such as, e.g., ethanol for use as a fuel or fuel additive), more preferably using a raw starch hydrolysis process. The processes described herein can be used, e.g., to increase the rate and/or yield of ethanol production. The addition of an effective amount of at least one esterase, which at least one esterase comprises a cutinase, optionally in combination with a lipase and/or a phospholipase can be used to raise the limit of ethanol tolerance of the fermenting microorganism and to thereby improve ethanol yield of the fermentation product by converting components, such as, oil from the corn substrate, to components useful the fermenting microorganism, e.g., unsaturated fatty acids and glycerol. As illustrated in the examples, the esterase treatment according to the present invention resulted in an ethanol yield increase.

Ethanol production processes generally involve the steps of milling, liquefaction, saccharification, fermentation and distillation. In the production of ethanol and other starch-based products, the raw material, such as whole grain, preferably corn, is milled in order to open up the structure and allow for further processing. Two processes are preferred according to the invention: wet milling and dry milling. Preferred for ethanol production is dry milling where the whole kernel is milled and used in the remaining part of the process. Wet milling may also be used and gives a good separation of germ and meal (starch granules and protein) and is with a few exceptions applied at locations where there is a parallel production of syrups. Both wet and dry milling processes are well known in the art.

In ethanol production, the fermenting organism is preferably yeast, which is applied to the mash. A preferred yeast is derived from *Saccharomyces* spp., more preferably, from Sac*charomyces cerevisiae.* In preferred embodiments, yeast is applied to the mash and the fermentation is ongoing for 24-96 hours, such as typically 35-60 hours. In preferred embodiments, the temperature is generally between 26-34°C, in particular about 32°C, and the pH is generally from pH 3-6, preferably around pH 4-5. Yeast cells are preferably applied in amounts of 10⁵ to 10¹², preferably from 10⁷ to 10¹⁰, especially 5x10⁷ viable yeast count per ml of fermentation broth. During the ethanol producing phase the yeast cell count should preferably be in the range from 10⁷ to 10¹⁰, especially around 2x10⁸. Further guidance in respect of using yeast for fermentation can be found in, e.g., "The alcohol Textbook" (Editors K. Jacques, T.P. Lyons and D.R.Kelsall, Nottingham University Press, United Kingdom 1999), which is hereby incorporated by reference.

Following fermentation, the mash may be distilled to extract the alcohol product (ethanol). In the case where the end product is ethanol, obtained according to the processes of the invention, it may be used as, e.g., fuel ethanol; drinking ethanol, i.e., potable neutral spirits; or industrial ethanol.

The esterases and other enzymes referenced herein may be derived or obtained from any suitable origin, including, bacterial, fungal, yeast or mammalian origin. The term "derived" or means in this context that the enzyme may have been isolated from an organism where it is present natively, i.e. the identity of the amino acid sequence of the enzyme are identical to a native enzyme. The term "derived" also means that the enzymes may have been produced recombinantly in a host organism, the recombinant produced enzyme having either an identity identical to a native enzyme or having a modified amino acid sequence, e.g. having one or more amino acids which are deleted, inserted and/or substituted, i.e., a recombinantly produced enzyme which is a mutant and/or a fragment of a native amino acid sequence or an enzyme produced by nucleic acid shuffling processes known in the art. Within the meaning of a native enzyme are included natural variants. Furthermore, the term "derived" includes enzymes produced synthetically by, e.g., peptide synthesis. The term "derived" also encompasses enzymes which have been modified e.g. by glycosylation, phosphorylation, or by other chemical modification, whether *in vivo* or *in vitro.* The term "obtained" in this context means that the enzyme has an amino acid sequence identical to a native enzyme. The term encompasses an enzyme that has been isolated from an organism where it is present natively, or one in which it has been expressed recombinantly in the same type of organism or another, or enzymes produced synthetically by, e.g., peptide synthesis. With respect to recombinantly produced enzymes the terms "obtained" and "derived" refers to the identity of the enzyme and not the identity of the host organism in which it is produced recombinantly.

The enzymes may also be purified. The term "purified" as used herein covers enzymes free from other components from the organism from which it is derived. The term "purified" also covers enzymes free from components from the native organism from which it is obtained. The enzymes may be purified, with only minor amounts of other proteins being present. The expression "other proteins" relate in particular to other enzymes. The term "purified" as used herein also refers to removal of other components, particularly other proteins and most particularly other enzymes present in the cell of origin of the enzyme of the invention. The enzyme may be "substantially pure," that is, free from other components from the organism in which it is produced, that is, for example, a host organism for recombinantly produced enzymes. In preferred embodiment, the enzymes are at least 75% (w/w) pure, more preferably at least 80%, at least 85%, at least 90%, at least 95%, at least 96%, at least 97%, at least 98%, or at least 99% pure. In another preferred embodiment, the enzyme is 100% pure.

The enzymes used in the present invention may be in any form (composition) suitable for use in the processes described herein, such as e.g. in the form of a dry powder or granulate, a non-dusting granulate, a liquid, a stabilized liquid, or a protected enzyme. Granulates may be produced, e.g., as disclosed in US Patent Nos. 4,106,991 and US 4,661,452, and may optionally be coated by methods known in the art. Liquid enzyme preparations may, for instance, be stabilized by adding stabilizers such as a sugar, a sugar alcohol or another polyol, lactic acid or another organic acid according to established methods. Protected enzymes may be prepared according to the method disclosed in EP 238,216.

When used in combination with processes or treatments which employ other enzymes, such as, amylases and glucoamylases used in liquefaction and/or saccharification processes, esterase compositions which do not inhibit these other enzymes are preferred, e.g., esterase compositions which do not contain or contain only minor amounts of calcium-binding compounds are preferred. Similarly, esterase compositions which do not inhibit fermentation processes are preferred, e.g., esterase compositions which do not contain or which contain only minor amounts of glycerol are preferred.

In accordance with another preferred embodiment, a fermentation stimulator may be used in combination with any of the enzymatic processes described herein to further improve the fermentation process, and in particular, the performance of the fermenting microorganism, such as, rate enhancement and ethanol yield. A "fermentation stimulator" refers to stimulators for growth of the fermenting microorganisms, in particular, yeast. Preferred fermentation stimulators for growth include vitamins and minerals. Examples of vitamins include multivitamins, biotin, pantothenate, nicotinic acid, meso-inositol, thiamine, pyridoxine, paraaminobenzoic acid, folic acid, riboflavin, and Vitamins A, B, C, D, and E. See, e.g., Alfenore et al., Improving ethanol production and viability of Saccharomyces cerevisia by a vitamin feeding strategy during fed-batch process," Springer-Verlag (2002), which is hereby incorporated by reference. Examples of minerals include minerals and mineral salts that can supply nutrients comprising P, K, Mg, S, Ca, Fe, Zn, Mn, and Cu.

### EXAMPLES

The embodiments shown in Examples 1, 4, 5, 7 and 9-14 do not fall within the scope of the claims.

### Example 1

The addition of an esterase, in particular, a lipase, was tested under anaerobic fermentation conditions analogous to an ethanol fermentation process. The experiment was conducted on dry milled ground yellow corn according to a simultaneous liquefaction-saccharification-fermentation (LSF) protocol. The experiment was carried out using glucoamylase (Spirizyme Plus, available from Novozymes A/S) in an amount of 0.0 (control); 0.8; 1.0, and 1.2 GAU/g DS and a fungal alpha amylase (SP288, available from Novozymes A/S) in an amount of 1.6 AFAU/g DS. The fermentations were compared to a fermentation with lipase (*Candida antarctica* lipase), added in an amount of 277 L/U g DS) in combination with 1.0 GAU glucoamylase (Spirizyme Plus) and 1.6 AFAU/g DS of a fungal alpha amylase (SP288). All fermentations were carried out at pH=5.0, 32°C for 72 hours. The process was monitored with time and analyzed for CO₂ weight loss, percent ethanol produced and theoretical conversion of the starch.

Table 1 illustrates the difference between fermentation with and without lipase. The lipase addition resulted in 20% ethanol yield in 72 hrs at 32°C along with a rate increase, which was better than treatment without lipase at any of the glucoamylase concentrations employed.

**Table 1**

| **GAU** | **Time of fermentation, h** | | | | | | | | | | |
|---|---|---|---|---|---|---|---|---|---|---|---|
| | **6** | **24** | **30** | **48** | | | **54** | **72** | | | |
| | CO₂, g | CO₂, g | CO₂, g | CO₂, g | Calculated ethanol, % v/v | Efficiency, % | CO₂, g | CO₂, g | Calculated ethanol, % v/v | HPLC ethanol, % v/v | Efficiency, % |
| 0.0 | 0.30 | 2.65 | 2.95 | 3.48 | 2.2 | 12.7 | 3.60 | 3.90 | 2.5 | 3.8 | 14.32 |
| 0.8 | 0.69 | 16.49 | 20.33 | 27.30 | 17.3 | 100.0 | 28.58 | 30.96 | 19.6 | 20.0 | 92.75 |
| 1.0 | 0.74 | 17.04 | 20.99 | 27.78 | 17.5 | 101.7 | 28.98 | 31.22 | 19.7 | 19.9 | 92.71 |
| 1.0 & lipase | 0.94 | 18.94 | 22.38 | 28.75 | 17.8 | 105.3 | 29.93 | 32.16 | 20.0 | 20.2 | 96.50 |
| 1.2 | 0.74 | 17.80 | 21.52 | 28.04 | 17.7 | 102.7 | 29.15 | 31.17 | 19.7 | 19.9 | 92.35 |

### Example 2

Example 2 was conducted on dry milled ground yellow corn according to a simultaneous liquefaction-saccharification-fermentation (LSF) protocol. The experiment was carried out using a glucoamylase (Spirizyme Plus, available from Novozymes A/S) in an amount of 0.8 GAU/g DS and a fungal alpha amylase (SP288, available from Novozymes A/S) in an amount of 2.6 AFAU/g DS for fermenting ethanol along with the addition of:
(A) lipase (LIPOLASE, available from Novozymes A/S),
(B) *Candida antarctica* lipase,
(C) cutinase (JC 492, available from Novozymes A/S)
(D) phospholipase (LECITASE available from Novozymes A/S),
(E) inactivated *Candida antarctica* lipase (inactivated by boiling for three hours), and
(F) a control (glucoamylase and amylase without esterase addition).

The esterases were added in an amount corresponding to 200 U/g DS. Fermentations were carried out at pH=5.0, 32 °C for several hours. The process was monitored with time and analyzed for CO₂ weight loss, percent ethanol produced and theoretical conversion of the starch.

As shown in Table 2, an increase in ethanol rate and yield was observed (along with a corresponding increase in fatty acids) for all of the active esterases tested. Efficiency was defined as an amount of ethanol produced per unit of solids. The best results were observed for *Candida antarctica* lipase.

**Table 2**

| **Lipase** | **Time of fermentation, h** | | | | | |
|---|---|---|---|---|---|---|
| | **24** | | **48** | | **72** | |
| | Calculated ethanol, % v/v | Efficiency, % | Calculated ethanol, % v/v | Efficiency, % | HPLC ethanol, % v/v | Efficiency, % |
| Control | 11.47 ± 0.05 | 52.19 ± 0.27 | 16.89 ± 0.12 | 81.91 ± 0.70 | 19.48 ± 0.04 | 89.37 ± 0.22 |
| Lipolase | 11.64 ± 0.21 | 53.07 ± 1.09 | 17.29 ± 0.31 | 84.26 ± 1.83 | 19.77 ± 0.12 | 91.04 ± 0.69 |
| Cutinase | 11.96 ± 0.09 | 55.20 ± 0.49 | 17.31 ± 0.07 | 85.19 ± 0.38 | 19.62 ± 0.08 | 91.18 ± 0.16 |
| Lecitase | 11.59 ± 0.04 | 53.50 ± 0.19 | 17.12 ± 0.08 | 84.35 ± 0.50 | 19.59 ± 0.08 | 91.19 ± 0.47 |
| *Candida antarctica* lipase inactive | 11.43 ± 0.08 | 52.88 ± 0.40 | 16.88 ± 0.15 | 83.18 ± 0.89 | 19.49 ± 0.04 | 90.89 ± 0.25 |
| *Candida antarctica lipase* | 12.25 ± 0.17 | 57.56 ± 1.14 | 17.97 ± 0.18 | 89.75 ± 1.08 | 20.34 ± 0.05 | 95.87 ± 0.30 |

### Example 3

Example 3 was carried out in the same manner as Example 2 except that *Candida antarctica* lipase was only partially inactivated (by boiling for 10 minutes.) As shown in Table 3, despite only partial inactivation, the *Candida antarctica* lipase still obtained a significant improvement.

**Table 3**

| **Lipase** | **Time of fermentation, h** | | | | | |
|---|---|---|---|---|---|---|
| | **24** | | **48** | | **72** | |
| | Calculated ethanol, % v/v | Efficiency, % | Calculated ethanol, % v/v | Efficiency, % | HPLC ethanol, % v/v | Efficiency, % |
| Control | 11.88 ± 0.10 | 53.46 ± 1.57 | 17.21 ± 0.12 | 83.63 ± 0.71 | 19.38 ± 0.13 | 88.62 ± 0.75 |
| Lipolase | 12.11±0.07 | 55.44 ± 0.34 | 17.65 ± 0.11 | 86.20 ± 0.58 | 19.86 ± 0.12 | 91.04 ± 0.59 |
| Cutinase | 12.39±0.07 | 57.39 ± 0.35 | 17.77 ± 0.18 | 87.59 ± 0.59 | 19.94 ± 0.10 | 92.54 ± 0.70 |
| Lecitase | 12.02 ± 0.15 | 55.65 ± 0.79 | 17.32 ± 0.13 | 85.58 ± 0.70 | 19.63 ± 0.08 | 91.32 ± 0.42 |
| *Candida antarctica* lipase briefly boiled | 12.01 ± 0.06 | 55.83 ± 0.34 | 17.68 ± 0.17 | 87.83 ± 1.01 | 20.10 ± 0.07 | 94.43 ± 0.37 |
| *Candida antarctica* lipase | 12.26 ± 0.11 | 57.48 ± 0.85 | 17.77 ± 0.16 | 88.74 ± 0.67 | 20.25 ± 0.07 | 95.10 ± 0.40 |

### Example 4

Example 4 was conducted on dry milled corn according to a simultaneous liquefaction-saccharification-fermentation (LSF) protocol. The experiment was carried out on ground yellow corn using a glucoamylase and alpha amylase for fermenting ethanol with and without lipase, as described in examples 2 and 3. Fermentations were carried out at pH=5.0, 32°C for several hours. In addition to *Candida antarctica* lipase and inactivated *Candida antarctica* lipase, feric sulfate and purified lipases from *Candida cylindracea* lipase and *Candida antarctica* lipase A, were also compared. Four times less of the purified lipase was used.

As shown in Table 4, significant improvements in ethanol yield at 48 hours were observed for all of the lipases, and in particular, the two purified lipases (*Candida cylindracea* lipase and *Candida antarctica* lipase A). *Candida antarctica* lipase A performed the best, showing an efficiency of 94.9% efficiency and 18% yield at 48 hours. *Candida cylindracea* lipase showed an 89.2% efficiency and a 17.8% yield at 48 hours. *Candida antarctica* lipase showed an 89% efficiency and a 17.6% yield at 48 hours. The purified lipases had the best performance even though four times less of the purified enzymes were used.

**Table 4**

| **Additive** | **Time of fermentation, h** | | | | | |
|---|---|---|---|---|---|---|
| | **24** | | **48** | | **72** | |
| | Calculated ethanol, % v/v | Efficiency, % | Calculated ethanol, % v/v | Efficiency, % | Calculated ethanol, % v/v | Efficiency, % |
| Control | 11.49 ± 0.15 | 52.33 ± 0.78 | 16.92 ± 0.23 | 82.05 ± 1.35 | 18.20 ± 0.26 | 89.67 ± 1.59 |
| *Candida antarctica* lipase inactive | 10.59 ± 0.16 | 49.33 ± 0.85 | 16.91 ± 0.30 | 84.73 ± 1.77 | 18.65 ± 0.40 | 95.49 ± 2.53 |
| *Candida antarctica* lipase 400 LU/g DS | 11.29 ± 0.24 | 51.87 ± 1.87 | 17.73 ± 0.37 | 89.03 ± 2.45 | 19.36 ± 0.59 | 99.19 ± 3.77 |
| Fe₂(SO₄)₃ | 11.58 ± 0.13 | 52.79 ± 0.67 | 16.87 ± 0.43 | 81.84 ± 2.57 | 18.82 ± 0.91 | 91.00 ± 2.45 |
| *Candida cylindracea* lipase, 100 LU/g DS | 11.46 ± 0.10 | 53.54 ± 0.10 | 17.75 ± 0.24 | 89.24 ± 3.19 | 19.06 ± 0.32 | 98.17 ± 1.38 |
| *Candida antarctica lipase A,* 100 LU/g DS | 11.58 ± 0.15 | 56.69 ± 0.15 | 17.98 ± 0.34 | 94.88 ± 2.16 | 19.83 ± 0.51 | 98.16 ± 3.14 |

### Example 5

Example 5 was conducted on dry milled corn according to a simultaneous liquefaction-saccharification-fermentation (LSF) protocol. The experiment was carried out on ground yellow corn using a glucoamylase and alpha amylase for fermenting ethanol with and without growth stimulators. Fermentations were carried out at pH=5.0, 32°C for several hours. Vitamins and minerals were added as follows:
A: Centrum (a multivitamin);
B: Vitamin E, Mg, Zn and Cr;
C: Vitamins A, C, D, E, and thiamine, riboflavin, nicotinic acid, folic acid, B-12, panthenate acid;
D: glucosamine and Vitamin C
E: Control, no addition

Table 5 illustrates that at 48 hours, further improvement can be obtained with vitamin and mineral additions.

**Table 5**

| **Vitamin** | **Time of fermentation, h** | |
|---|---|---|
| | **48** | **72** |
| | Calculated ethanol, % v/v | HPLC ethanol, % v/v |
| Control | 16.54 ± 0.23 | 19.63 ± 0.06 |
| A | 16.65 ± 0.18 | 19.34 ± 0.19 |
| B | 16.77 ± 0.07 | 19.28 ± 0.04 |
| C | 16.94 ± 0.11 | 19.33 ± 0.05 |
| D | 16.81 ± 0.14 | 19.29 ± 0.02 |

### Example 6

In Examples 6-13, a 25-ml disposable fermentor was designed for the experiment. The fermentor was made of 50-ml polypropylene conical tube available from Becton Dickinson Labware, USA. Fermentation lock was made of disposable polyethylene transfer pipet available from Fisher Scientific, USA attached to a 5-ml syringe. The syringe was equipped with 0.45-µm Whatman PVDF syringe filter available from Fisher Scientific, USA.

With the 25-ml disposable fermentor it was possible to run many samples in the same water bath so as to eliminate common experimental error caused by temperature fluctuation of water. No sample transfer was required after completion of fermentation. Samples to be analyzed were also able to be spun down in the same tubes which substantially reduced the time of analysis.

Yeast (Ethanol Red yeast, 36% DS) was propagated with maltodextrin aerobically at 500 rpm and 32.2° C for 16 h. A corn slurry was prepared by mixing ground corn (2-mm screen) and water followed by pH adjustment with inorganic acid.

Amylase (1.1 AFAU/g DS SP 288), glucoamylase (1.5 GAU/g DS Spirizyme Plus), and yeast were introduced into the slurry immediately before filling the fermentors with the resulting medium.

The following esterase enzymes were added in an amount corresponding to 200 U/g DS before filling the fermentors:
A) Lipolase 100T (lipase available from Novozymes),
B) *Candida antarctica* B lipase (CALB)),
C) Lecitase 10 L (phospholipase, available from Novozymes)
D) JC 492 (cutinase, available from Novozymes A/S)

Fermentation was carried in the 25-ml fermentors at 32.2° C without mixing for 72 h. CO₂ weight loss was taken in 24, 48, and 72 h. When fermentation was completed, samples were spun down at 3,000 rpm at 20° C for 15 min, forced through the 0.45-µm filter and about 2 ml of beer fraction was withdrawn and analyzed by HPLC.

A control was preformed as described above, without the addition of an esterase.

As shown in Tables 6, all esterases tested significantly increased ethanol yield. The 24-h and 48-h data were calculated based on CO₂ weight loss, and 72-h data were obtained by HPLC.

The efficiency of the process was also significantly increased by purified esterase treatment and, in particular, reached 95.1% in the case of *Candida antarctica* B lipase, as shown in Table 7, below.

**Table 6**

| **Esterase** | **Time of fermentation, h** | | |
|---|---|---|---|
| | **24** | **48** | **72** |
| | Calculated ethanol, % v/v | Calculated ethanol, % v/v | HPLC ethanol, % v/v |
| Control | 11.88 ± 0.10 | 17.21 ± 0.12 | 19.38 ± 0.13 |
| Lecitase 10L | 12.02 ± 0.15 | 17.32 ± 0.13 | 19.63 ± 0.08 |
| Lipolase 100T | 12.11 ± 0.07 | 17.65 ± 0.11 | 19.86 ± 0.12 |
| Cutinase JC 492 | 12.39 ± 0.07 | 17.77 ± 0.18 | 19.94 ± 0.10 |
| CALB | 12.26 ± 0.11 | 17.77 ± 0.16 | 20.25 ± 0.07 |

**Table 7**

| **Esterase** | **Efficiency, %** | **CL, %** |
|---|---|---|
| Control | 88.62 | 0.75 |
| Cutinase | 92.54 | 0.70 |
| Phospholipase | 91.32 | 0.42 |
| Lipolase | 91.64 | 0.59 |
| CALB | 95.10 | 0.40 |

### Example 7

Example 7 was carried out using Alltech SuperStart yeast, 36% DS, 1 GAU/g DS Spirizyme Fuel, 0.8 AFAU/g DS SP 288. Yeast was propagated with maltodextrin. A comparison was made between *Candida Antarctica* lipase A, added at .8 LU/g DS, 4 LU/g DS and 20 LU/g DS and protease (GC 106, available from Genencor) added at .007% and .014%. Fermentation was carried out in 25-ml fermentors at 32.2° C without mixing for 72 h

As shown in Table 8, both lipase and protease had an effect on ethanol yield. However, the effect of lipase used at .8 LU/g DS, 4 LU/g DS and 20 LU/g DS was significantly higher than the effect of protease alone used at .007% DS and .014% DS.

**Table 8**

| **Treatment** | **HPLC ethanol, % v/v** |
|---|---|
| Control | 12.66 ± 0.27 |
| Lipase, 0.8 LU/g DS | 14.64 ± 0.21 |
| Lipase, 4 LU/g DS | 14.66 ± 0.07 |
| Lipase, 20 LU/g DS | 13.81 ± 0.21 |
| Lipase, 100 LU/g DS | 11.00 ± 0.18 |
| Protease, 0.007% DS | 12.61 ± 0.12 |
| Protease, 0.014% DS | 13.50 ± 0.17 |

### Example 8

This experiment was carried out using the same experimental conditions and enzyme concentrations described in Example 6, with the exception that protease (Novozyme 50006, 0.007% DS) was also added to the fermentation medium with an increased sample volume.

To eliminate the possibility that a substance in the CALB formulation was contributing to the effect obtained, inactivated CALB was also used. A control was prepared by inactivating CALB by boiling for 3 hours. Upon completion of boiling, the volume was adjusted to the initial value with DI water, and sample was spun down at 3,000 rpm for 20 min. The liquid layer was forced through the 0.45-µm filter. The enzyme was thereby completely inactivated.

The experimental data is shown in Table 9, below. The value for the efficiency ranged from 91.04% to 95.87%, with the lipase CALB showing the best results. There was no significant difference between ethanol yield of control samples and ethanol yield of samples treated with inactivated CALB (results are not shown).

### Example 9

Example 9 was carried out using the same experimental conditions described in Example 7, except the protease (Novozyme 50006, 0.007% DS) was added in the propogate.

In this experiment, purified lipase from *Candida cylindracea* and purified lipase A from *Candida Antarctica* (CALA) were added in amounts of 100 LU/g DS and purified lipase B from *Candida Antarctica* (CALB) was added in an amount of 400 LU/g DS. As shown in Table 9, these lipases also had a substantial effect on both ethanol yield and efficiency.

### Example 10

In this example, Lipolase 100 T and purified Lipolase (both available from Novozymes A/S) were compared. The Experimental conditions were as follows: Ethanol Red yeast, 36% DS, 1.5 GAU/g DS Spirizyme Fuel, 0.8 AFAU/g DS SP 288. Yeast was propagated with maltodextrin and protease. Fermentation was carried out in 25-ml fermentors at 32.2° C without mixing for 72 h. A control was run as described, without the addition of lipase.

As shown in Table 9, these esterase also had a substantial effect on ethanol production.

### Example 11

An acidic lipase (Amano G, available from Amano Enzyme, USA) and the purified *C. cylindracea* lipase were compared. The experimental conditions were as follows: Ethanol Red yeast, 36% DS, 2.0 GAU/g DS Spirizyme Fuel, 0.8 AFAU/g DS SP 288. Yeast was propagated with maltodextrin and protease (Novozym 50006). Fermentation was carried out in 25-ml fermentors at 32.2° C without mixing for 72 h. There was 0.08% DS protease (Novozym 50006) in the fermenting medium.

As shown in Table 9, both the acidic lipase and purified *C. cylindracea* lipase showed an improvement over the control.

### Example 12

In this experiment, an acidic lipase (Amano G, available from Amano Enzyme, USA), purified phospholipase (Lecitase Ultra, available from Novozymes) and purified lipase (Lipex, available from Novozymes) were compared. The experimental conditions were as follows: Ethanol Red yeast, 36% DS, 2.0 GAU/g DS Spirizyme Fuel, 0.8 AFAU/g DS SP 288. Yeast was propagated with maltodextrin and Novozym 50006 protease. Fermentation was carried out in 25-ml fermentors at 32.2° C without mixing for 72 h. 0.02% DS Novozym 50006 was added to the fermenting medium.

As shown in Table 9, these esterases also resulted in an improvement over the control.

### Example 13

A 64-hour fermentation with acidic Amano G (available from Amano Enzyme, USA), purified phospholipase (Lecitase Ultra, available from Novozymes) and purified lipase (Lipex available from Novozymes) and lipase (Lipolase 100T, available from Novozymes) were compared. The condition of the experiment, were as follows: Ethanol Red yeast, 36% DS, 2.0 GAU/g DS Spirizyme Fuel, 0.8 AFAU/g DS SP 288. Yeast was propagated with maltodextrin and Novozym 50006.

Fermentation was carried out in 25-ml fermentors at 32.2° C without mixing for 72 h. 0.02% DS Novozym 50006 was added to the fermenting medium. As shown in Table 9, these esterases also had a significant impact on ethanol yield.

**Table 9. Effect of esterases on ethanol yield and efficiency.**

| Enzyme | Activity, LU/g DS | Ethanol,% v/v in beer | Change, % v/v *vs.* control | Efficiency, % | Change, % v/v vs. control | n | Example |
|---|---|---|---|---|---|---|---|
| Acidic lipase | 1 | 21.12 | +0.68 | 98.92 | +3.85 | 11 | Example 11 |
| Acidic lipase | 1 | 20.92 | +0.14 | 98.00 | +0.83 | 12 | Example 12 |
| Acidic lipase | 5 | 21.00 | +0.56 | 98.25 | +3.18 | 11 | Example 11 |
| Acidic lipase | 10 | 21.16 | +0.72 | 99.15 | +4.08 | 12 | Example 11 |
| Acidic lipase | 10 | 20.98 | +0.20 | 98.31 | +1.14 | 11 | Example 12 |
| Acidic lipase | 10 | 19.99 | +0.18 | 92.53 | +0.90 | 11 | Example 13 |
| Acidic lipase | 100 | 21.34 | +0.90 | 100.53 | +5.46 | 9 | Example 11 |
| Acidic lipase | 100 | 20.99 | +0.21 | 98.42 | +1.25 | 12 | Example 12 |
| CALA (purified) | 100 | 19.83 | +1.63 | 98.16 | +8.49 | 11 | Example 9 |
| *C. cylindracea* (purified) | 1 | 20.85 | +0.41 | 97.36 | +2.29 | 12 | Example 11 |
| *C. cylindracea* (purified) | 10 | 20.60 | +0.16 | 95.96 | +0.89 | 12 | Example 11 |
| *C. cylindracea* (purified) | 100 | 19.06 | +0.86 | 98.17 | +8.50 | 11 | Example 9 |
| Cutinase JC 492 | 200 | 19.94 | +0.56 | 92.54 | +3.92 | 12 | Example 6 |
| Cutinase JC 492 | 200 | 19.62 | +0.14 | 91.18 | +1.81 | 14 | Example 8 |
| Lecitase 10 L | 200 | 19.63 | +0.25 | 91.32 | +2.70 | 12 | Example 6 |
| Lecitase 10 L | 200 | 19.59 | +0.11 | 91.19 | +1.82 | 12 | Example 8 |
| Lecitase Ultra (purified) | 1 | 21.05 | +0.27 | 98.74 | +1.57 | 12 | Example 12 |
| Lecitase Ultra (purified) | 1 | 20.33 | +0.52 | 94.50 | +2.87 | 11 | Example 13 |
| Lecitase Ultra (purified) | 10 | 21.28 | +0.50 | 100.14 | +2.97 | 11 | Example 12 |
| Lecitase Ultra (purified) | 10 | 19.89 | +0.08 | 91.98 | +0.35 | 12 | Example 13 |
| Lipex (purified) | 1 | 21.30 | +0.52 | 100.22 | +3.03 | 12 | Example 12 |
| Lipex (purified) | 10 | 21.08 | +0.30 | 98.95 | +1.78 | 12 | Example 12 |
| Lipex (purified) | 10 | 20.07 | +0.26 | 92.99 | +1.36 | 10 | Example 13 |
| Lipolase (purified) | 1 | 18.41 | +0.31 | 84.00 | +1.00 | 12 | Example 10 |
| Lipolase (purified) | 10 | 18.62 | +0.52 | 83.49 | +0.49 | 8 | Example 10 |
| Lipolase (purified) | 100 | 18.24 | +0.14 | 83.57 | +0.57 | 10 | Example 10 |
| Lipolase 100 T | 1 | 18.33 | +0.23 | 84.87 | +1.87 | 9 | Example 10 |
| Lipolase 100 T | 5 | 18.41 | +0.31 | 83.84 | +1.63 | 9 | Example 10 |
| Lipolase 100 T | 10 | 17.99 | -0.11 | 81.35 | -1.65 | 11 | Example 10 |
| Lipolase 100 T | 10 | 19.89 | +0.08 | 91.93 | +0.30 | 11 | Example 13 |
| Lipolase 100 T | 100 | 18.50 | +0.40 | 84.02 | +1.02 | 9 | Example 10 |
| Lipolase 100 T | 200 | 19.86 | +0.48 | 91.64 | +3.02 | 12 | Example 6 |
| Lipolase 100 T | 200 | 19.77 | +0.29 | 91.04 | +1.67 | 10 | Example 8 |
| CALB | 200 | 20.25 | +0.87 | 95.10 | +6.48 | 12 | Example 6 |
| CALB | 200 | 20.34 | +0.86 | 95.87 | +6.50 | 11 | Example 8 |
| CALB | 400 | 19.36 | +1.16 | 99.19 | +9.52 | 9 | Example 9 |
| CALA | 0.8 | 14.64 | +1.98 | 64.00 | +8.34 | 12 | Example 7 |
| CALA | 4 | 14.66 | +2.00 | 64.04 | +8.40 | 12 | Example 7 |
| CALA | 20 | 13.81 | +1.15 | 59.80 | +4.14 | 12 | Example 7 |

### EXAMPLE 14

The addition of a laccase was tested under anaerobic fermentation conditions analogous to ethanol fermenting experiments. The experiment was conducted on dry milled yellow corn according to a simultaneous liquefaction-saccharification-fermentation (LSF) protocol. The experiment was carried out using a glucoamylase (Spirizyme plus, available from Novozymes A/S) for fermenting ethanol, with and without laccase. Fermentations were carried out at pH=5.0, 32°C for several hour. The process was monitored with time and analyzed for CO₂ weight loss, percent ethanol produced and theoretical conversion of the starch. As shown in Table 10, the addition of laccase also improved ethanol yield.

**Table 10**

| **Treatment** | **HPLC ethanol, % v/v** | | | | | |
|---|---|---|---|---|---|---|
| | **7 h** | **23 h** | **29 h** | **47 h** | **55 h** | **119 h** |
| 0.7 GAU/g DS | 2.36 | 9.29 | 10.55 | 12.38 | 13.13 | 16.73 |
| 0.7 GAU/g DS & 0.1 M NaCl | 1.88 | 7.91 | 8.78 | 10.30 | 10.94 | 14.61 |
| 0.9 GAU/g DS | 2.24 | 9.63 | 10.92 | 12.92 | 13.66 | 18.05 |
| 0.9 GAU/g DS & NZ 525, 0.5% DS | 2.42 | 9.44 | 10.58 | 12.63 | 13.39 | 18.28 |
| 1.0 GAU/g DS | 2.07 | 9.29 | 10.21 | 12.27 | 13.06 | 17.83 |
| 1.0 GAU/g DS & laccase, 0.2% DS | 2.89 | 10.87 | 11.99 | 13.82 | 14.41 | 18.86 |

## Claims

1. A method for producing a fermentation product, which method comprises a fermentation step, comprising contacting a fermenting microorganism or fermentation media used in the fermentation step with at least one esterase enzyme, wherein said at least one esterase comprises a cutinase.

2. The method of claim 1, further comprising a lipase, a phospholipase or combinations thereof.

3. The method of any of claims 1 or 2, wherein said microorganism is a yeast.

4. The method of any of claims 1 to 3, wherein said fermentation product is ethanol.

5. The method of any of claims 1 to 4, wherein said fermentation step is part of a simultaneous saccharification and fermentation process.

6. The method of any of claims 1 to 5, wherein said fermentation step is part of a raw starch hydrolysis process.

7. The method of any of claims 1 to 6, wherein the fermentation step is carried out in the presence a glucoamylase and an amylase.

8. The method of any of claims 1 to 7, wherein the fermentation step is part of a dry milling process or a wet milling process.

9. The method of any of claims 1 to 8, wherein the raw material for milling process is a starch-containing raw material.

10. The method of any of claims 1 to 9, wherein the raw material for milling process is a selected from the group consisting of corn, wheat, barley, or milo.

11. The method of any of claims 1 to 10, wherein the fermentation media comprises de-germed cereal grain.

12. The method of any of claims 1 to 11, wherein the fermentation media comprises de-germed corn.

13. The method of any of claims 1 to 12, further comprising contacting a fermenting microorganism or fermentation media with an enzymes selected from the consisting of proteases, phytases, and cellulases.

14. A method for producing ethanol, comprising
(a) milling whole grains;
(b) liquefying the product of step (a);
(c) saccharifying the liquefied material;
(d) fermenting the saccharified material using a microorganism, wherein the fermentation process further comprises contacting the fermenting microorganism or media containing the fermenting microorganism with at least one esterase enzyme, wherein said at least one esterase comprises a cutinase.

15. The method of claim 14, further comprising distilling the fermented material.

16. The method of any of claims 14 or 15, wherein said method is a simultaneous liquefaction and saccharification process or a simultaneous liquefaction, saccharification and fermentation process.

17. The method of any of claims 14 to 16, wherein said method comprises adding an alpha-amylase, a glucoamylase to a fermentation media comprising a fermentation microorganism.

18. The method of any of claims 14 to 17, furthermore comprising a lipase, a phospholipase or combinations thereof.

19. The method of any of claims 14 to 18, wherein said microorganism is a yeast.

## Patentansprüche

1. Verfahren zur Herstellung eines Fermentationsprodukts, wobei das Verfahren einen Fermentationsschritt einschließt, der das Zusammenbringen eines fermentierenden Mikroorganismus oder eines Fermentationsmediums, das bei dem Fermentationsschritt eingesetzt wird, mit mindestens einem Esteraseenzym umfasst, wobei die mindestens eine Esterase eine Cutinase umfasst.

2. Verfahren nach Anspruch 1, das außerdem eine Lipase, eine Phospholipase oder Kombinationen davon umfasst.

3. Verfahren nach einem der Ansprüche 1 oder 2, wobei der Mikroorganismus eine Hefe ist.

4. Verfahren nach einem der Ansprüche 1 bis 3, wobei das Fermentationsprodukt Ethanol ist.

5. Verfahren nach einem der Ansprüche 1 bis 4, wobei der Fermentationsschritt Teil eines gleichzeitigen Saccharifizierungs- und Fermentationsverfahrens ist.

6. Verfahren nach einem der Ansprüche 1 bis 5, wobei der Fermentationsschritt Teil eines Rohstärke-Hydrolyseverfahrens ist.

7. Verfahren nach einem der Ansprüche 1 bis 6, wobei der Fermentationsschritt in Gegenwart einer Glucoamylase und einer Amylase durchgeführt wird.

8. Verfahren nach einem der Ansprüche 1 bis 7, wobei der Fermentationsschritt Teil eines Trockenmahlverfahrens oder eines Nassmahlverfahrens ist.

9. Verfahren nach einem der Ansprüche 1 bis 8, wobei das Rohmaterial für das Mahlverfahren ein Stärke enthaltendes Rohmaterial ist.

10. Verfahren nach einem der Ansprüche 1 bis 9, wobei das Rohmaterial für das Mahlverfahren aus der Gruppe ausgewählt ist, die aus Mais, Weizen, Gerste oder Hirse besteht.

11. Verfahren nach einem der Ansprüche 1 bis 10, wobei das Fermentationsmedium entkeimte Getreidekörner umfasst.

12. Verfahren nach einem der Ansprüche 1 bis 11, wobei das Fermentationsmedium entkeimten Mais umfasst.

13. Verfahren nach einem der Ansprüche 1 bis 12, das weiterhin das Zusammenbringen eines fermentierenden Mikroorganismus oder eines Fermentationsmediums mit einem Enzym, das aus der Gruppe ausgewählt ist, die aus Proteasen, Phytasen und Cellulasen besteht, umfasst.

14. Verfahren zur Herstellung von Ethynol, umfassend
(a) Mahlen von ganzen Getreidekörnern;
(b) Verflüssigen des Produktes von Schritt (a);
(c) Saccharifizieren des verflüssigten Materials;
(d) Fermentieren des verflüssigten Materials unter Verwendung eines Mikroorganismus, wobei das Fermentationsverfahren außerdem das Zusammenbringen des fermentierenden Mikroorganismus oder des Fermentationsmediums, das den fermentierenden Mikroorganismus enthält, mit mindestens einem Esteraseenzym umfasst, wobei die mindestens eine Esterase eine Cutinase umfasst.

15. Verfahren nach Anspruch 14, das außerdem das Destillieren des fermentierten Materials umfasst.

16. Verfahren nach einem der Ansprüche 14 oder 15, wobei das Verfahren ein gleichzeitiges Verflüssigungs- und Saccharifizierungsverfahren oder ein gleichzeitiges Verflüssigungs-, Saccharifizierungs- und Fermentationsverfahren ist.

17. Verfahren nach einem der Ansprüche 14 bis 16, wobei das Verfahren die Zugabe einer alpha-Amylase, einer Glucoamylase zu einem Fermentationsmedium, das einen Fermentationsmikroorganismus umfasst, umfasst.

18. Verfahren nach einem der Ansprüche 14 bis 17, das außerdem eine Lipase, eine Phospholipase oder Kombinationen davon umfasst.

19. Verfahren nach einem der Ansprüche 14 bis 18, wobei der Mikroorganismus eine Hefe ist.

## Revendications

1. Méthode pour produire un produit de fermentation, la méthode comprenant une étape de fermentation comprenant la mise en contact d'un microorganisme fermenteur ou d'un milieu de fermentation utilisé dans l'étape de fermentation avec au moins une enzyme estérase, dans laquelle ladite au moins une estérase comprend une cutinase.

2. Méthode selon la revendication 1, comprenant en outre une lipase, une phospholipase ou des combinaisons de celles-ci.

3. Méthode selon l'une quelconque des revendications 1 et 2, dans laquelle ledit microorganisme est une levure.

4. Méthode selon l'une quelconque des revendications 1 à 3, dans laquelle ledit produit de fermentation est l'éthanol.

5. Méthode selon l'une quelconque des revendications 1 à 4, dans laquelle ladite étape de fermentation fait partie d'un procédé de saccharification et de fermentation simultanées.

6. Méthode selon l'une quelconque des revendications 1 à 5, dans laquelle ladite étape de fermentation fait partie d'un procédé d'hydrolyse d'amidon brut.

7. Méthode selon l'une quelconque des revendications 1 à 6, dans laquelle ladite étape de fermentation est effectuée en présence d'une glucoamylase et d'une amylase.

8. Méthode selon l'une quelconque des revendications 1 à 7, dans laquelle ladite étape de fermentation fait partie d'un procédé de mouture sèche ou humide.

9. Méthode selon l'une quelconque des revendications 1 à 8, dans laquelle la matière première pour le procédé de mouture est une matière première contenant de l'amidon.

10. Méthode selon l'une quelconque des revendications 1 à 9, dans laquelle la matière première pour le procédé de mouture est sélectionnée parmi le groupe constitué du maïs, du blé, de l'orge et du sorgho.

11. Méthode selon l'une quelconque des revendications 1 à 10, dans laquelle le milieu de fermentation comprend des grains de céréale dégermés.

12. Méthode selon l'une quelconque des revendications 1 à 11, dans laquelle le milieu de fermentation comprend du maïs dégermé.

13. Méthode selon l'une quelconque des revendications 1 à 12, comprenant en outre la mise en contact d'un microorganisme fermentant ou d'un milieu de fermentation avec une enzyme sélectionnée parmi le groupe constitué des protéases, des phytases et des cellulases.

14. Méthode pour produire de l'éthanol comprenant
(a) la mouture de grains entiers ;
(b) la liquéfaction du produit de l'étape (a) ;
(c) la saccharification du matériau liquéfié ;
(d) la fermentation du matériau saccharifié en utilisant un microorganisme, dans laquelle le procédé de fermentation comprend en outre la mise en contact d'un microorganisme fermentant ou d'un milieu contenant le microorganisme fermentant avec au moins une enzyme estérase, dans laquelle ladite au moins une estérase comprend une cutinase.

15. Méthode selon la revendication 14, comprenant en outre la distillation du matériau fermenté.

16. Méthode selon l'une quelconque des revendications 14 et 15, dans laquelle ladite méthode est un procédé de liquéfaction et de saccharification simultanées ou un procédé de liquéfaction, de saccharification et de fermentation simultanées.

17. Méthode selon l'une quelconque des revendications 14 à 16, dans laquelle ladite méthode comprend l'ajout d'une alpha-amylase, d'une glucoamylase à un milieu de fermentation comprenant un microorganisme de fermentation.

18. Méthode selon l'une quelconque des revendications 14 à 17, comprenant en outre une lipase, une phospholipase ou des combinaisons de celles-ci.

19. Méthode selon l'une quelconque des revendications 14 à 18, dans laquelle ledit microorganisme est une levure.
